# EUROPEAN PATENT APPLICATION

(11) **EP 1 284 157 A1**
(43) Date of publication of application: **19.02.2003**
(21) Application number: 02015636.0
(22) Date of filing: 16.07.2002
(51) Int. Cl.: B01J 8/00, B01J 8/02, C07C 37/20

(54) **Reactor for running the reaction of bisphenol A obtaining**

(30) Priority: 07.08.2001 PL 34907901
(71) Applicant: INSTYTUT CIEZKIEJ SYNTEZY ORGANICZNEJ "BLACHOWNIA", 47-225 Kedzierzyn-Kozle (PL); Petro Carbo Chem S.A., 44-117 Gliwice (PL)
(72) Inventor: Kiedik, Maciej, Kedzierzyn-Kozle (PL); Kolt, Jozef, Zabrze (PL); Pokorska, Zofia, Kedzierzyn-Kozle (PL); Szymanski, Kazimierz, Kedzierzyn-Kozle (PL); Kosciuk, Ryszard, Kedzierzyn-Kozle (PL); Rzodeczko, Anna, Kedzierzyn-Kozle (PL); Matyja, Stanislaw, Kedzierzyn-Kozle (PL); Rdesinska-Cwik, Teresa, Kedzierzyn-Kozle (PL); Kaledkowska, Malgorzata, Kedzierzyn-Kozle (PL)
(74) Representative: Patentanwälte Zellentin & Partner

(57) **Abstract**

This invention relates to a reactor for running the reaction producing bisphenol A from phenol and acetone at the presence of strongly acidic cation exchanger as a catalyst with a reaction promoter chemically bonded to it. The reaction mixture is passed upwards through the catalyst bed. The reactor has been provided with four filtration systems which have been inserted through nozzles in the reactor heads. Two of those systems **(2)** and **(3)** are operated in the upper part of the catalyst bed and they are protected against sideways movements, while the other two systems **(1)** and **(4)** are located close to the bottom and top heads of the reactor, respectively. It is preferable when filtration slotnozzles **(5)** are employed as filtration elements and their number has been selected to make at least one nozzle fall to 1 m² of the reactor horizontal section. Moreover, an inert granular material with the specific gravity under reaction conditions below that of the reaction mixture or a weak alkaline anion exchanger with the specific gravity below that of the catalyst should preferably be added to the catalyst.

## Description

The subject of this invention is a reactor for running the reaction of obtaining bisphenol A which is a raw material for epoxy and polycarbonate resins production. The invention includes the reactor which is employed for bisphenol A obtaining from phenol and acetone at the presence of strongly acid cation exchanger containing chemically bonded reaction promoter as a catalyst, with the reaction mixture passing through the reactor upwards, the said mixture obtained by mixing phenol and acetone together with a stream of the reaction mixture circulating in the reaction system, withdrawn from the catalyst bed, cooled down and recycled to be fed to the reactor.

Application of highly active promoters of the reaction of phenol and acetone, which are'firmly bonded to strongly acidic cation exchanger used as a catalyst in the bisphenol A manufacturing process improved the yield of the product obtained from the cation exchanger volume unit for more than 2.5 times. On the other hand, equivalent difficulties were met in taking increased heat of the reaction away from the catalyst bed without detrimental temperature increase in the reactor.

The European patent EP 0754666 teaches that favourable reaction conditions and lower temperature rise in the catalyst bed are available when two or three reactors are employed connected in series instead of one reactor and the reaction mixture is subjected to cooling between the said reactors. However, such arrangement considerably increases investment costs and costs of operation of the reaction system.

The principal and commonly accepted method for removal of heat evolved by the reaction of phenol and acetone in the catalyst bed is use of huge excess (about 10:1) of phenol in the reaction mixture in relation to the stoichiometric phenol/acetone molar ratio which is 2:1. Similarly, the US patent 5315042 reveals a method based on control of increase of bisphenol A concentration in the reaction mixture. Both these known methods result in higher energy consumption necessary for separation of the product from the reaction mixture. There is also known a method for removal of heat of the phenol/acetone reaction from the catalyst bed and thus for providing lower temperature rise, which is based on partial re-circulation of the reaction effluent stream to the reactor inlet after cooling it down.

Performing the reaction of phenol and acetone when the reaction mixture flows upwards through the promoted catalyst involves some difficulties at higher rates of the reaction mixture flow through the catalyst bed. Depending upon the conditions of reaction running and disintegration of the bed caused by the flowing reaction mixture, the "top" layer of the catalyst bed may "float" at various levels inside the reactor. The phenol/acetone molar ratio in the reaction feed mixture, which is usually about 10:1, usually practised in industry, allows to control temperature increase of the reaction mixture in the catalyst bed at about 20 K with the rise of bisphenol A concentration in the reaction mixture for about 10% by weight for one pass of the reaction mixture through the catalyst bed. Such temperature increase of the reaction mixture in the reactor makes achievement of properly high reaction selectivity impossible.

It is assumed that high selectivity of the reaction of phenol with acetone and accepted high promoter life are available if the temperature increase in the reactor catalyst bed does not exceed 5 K and the reaction temperature is slowly increased over the catalyst operating cycle starting from the temperature below 328 K.

Unexpectedly it was found out that considerably better selectivity of the reaction of phenol and acetone, with the reaction mixture passing upwards through the catalyst bed in the form of strongly acidic cation exchanger with chemically bonded reaction promoter, was achieved when the reactor construction made possible to control the reaction heat removal by withdrawing the reaction mixture stream from the top half of the reaction bed and from above the bed, when the catalyst bed disintegration degree was controlled by the flow rate of the reaction mixture. The reactor design which enables such reaction heat removal also enables achievement of extended time of the reaction promoter high activity and higher increase of bisphenol A concentration in the reaction mixture with no excessive temperature rise. Due to higher concentration of bisphenol A in the reactor effluent stream, the consumption of heat energy needed for separation of the final product is considerably reduced.

The new design of the reactor fort bisphenol A production allows to run the process more favourably than it was possible in case of previous reactors constructions.

The essence of the invention consists in the fact that the reactor in the form of a vertical, cyllindrical pressure vessel, wherein the height-to-diameter ratio is from 2:1 to 5:1, has been provided with four systems of filtration elements which have been inserted through nozzles in the reactor heads. Under operating conditions, two such systems are in the upper half of the catalyst bed and the remaining two systems are located at the bottom and the top heads of the reactor vessel, respectively.

It is preferable when the filtration elements are slot-type filtration nozzles.

It is preferable when the filtration elements operated in the upper part of the catalyst bed are protected against sideways movements by means of sleeves which loosely embrace them, being truss joints of horizontal framework linking together all the sleeves and fixed to the reactor walls in numerous points spaced along the reactor perimeter.

It is preferable when the filtration elements are arranged uniformly over the reactor cross-sections.

It is preferable when two filtration systems of slot-type filtration nozzles operated in the upper part of the catalyst bed are composed of filtration elements being parts of pipes inserted to the reactor through nozzles in the reactor head, with two filtration elements installed on every pipe: one at the pipe tip and the other one up to 2m away from the first element.

It is preferable when the reactor height-to-diameter ratio is from 2.5:1 to 3.5:1.

It is preferable when the number of filtration-slot nozzles operated in the upper part of the catalyst bed is such that at least one nozzle falls to 1 m² of the reactor horizontal cross-section.

It is further preferable when inert granular material is added to the catalyst bed, with its specific gravity under reaction conditions below that of the catalyst, and in particular below that of the reaction mixture. The volume of that additional material should be from 2% to 10% by volume of the catalyst bed.

It is yet preferable when weak alkaline anion exchanger is added to the catalyst bed, with its specific gravity under reaction conditions below that of the catalyst. The volume of said anion exchanger should preferably be from 5% to 15% by volume of the catalyst bed.

Running the reaction in the reactor according to this invention provides better selectivity of the reaction of phenol and acetone to bisphenol A and it involves extending of a period of high activity of the reaction promoter. Moreover, it enables achievement of higher increase of bisphenol A concentration in the reaction mixture without excessive increase of the temperature in the reactor, in comparison to the known methods for reaction running and the reactors employed up to now.

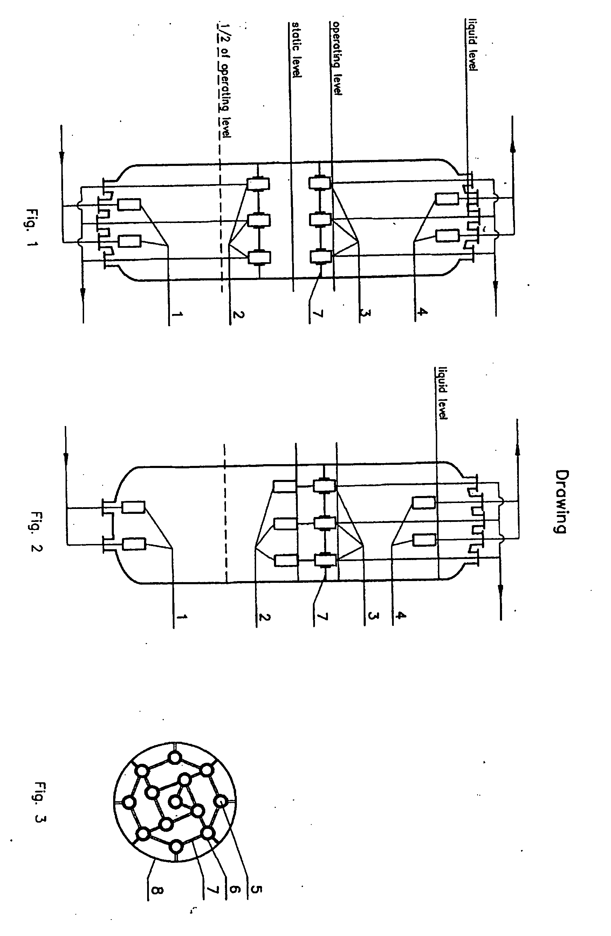

The reactor presented on Fig.1 illustrates the reactor design wherein the filtration elements of one of the filtration systems operated in the upper part of the catalyst bed have been introduced through nozzles in the reactor bottom head, while the filtration elements of the other filtration system have been introduced through nozzles in the reactor top head. Fig.2 illustrates construction of the reactor version in which the filtration elements of both filtration systems have been introduced through the same nozzles in one reactor head. This makes the reactor design and accessories simpler and hence reduces the costs of construction and operation of the reactor. To make the illustration easier, Fig. 1 and Fig.2 present only a few filtration elements of each of four filtration systems of the reactor. Fig.3 presents the reactor cross-section with the truss structure and its sleeves loosely embracing several filtration elements in order to prevent their sideways movements.

The reactor of the height-to-diameter ratio 3:1 has been equipped with a system of filtration nozzles **1** at its bottom head to feed the raw materials and the recycled reaction mixture, with a system of filtration nozzles **2** and **3** operated in the upper part of the catalyst bed and used to withdraw the reaction mixture circulating in the reaction system, and with a system of filtration nozzles **4** located above the catalyst to evacuate the reactor effluent stream. The filtration nozzles **5** of the filtration systems **2** and **3** have been protected from sideways movements with sleeves **6** loosely embracing them, forming truss joints of a horizontal framework **7**, linking together all the sleeves and fixed to the reactor walls **8** in numerous points spaced along the reactor circumference.

The reactor has been designed to be operated with a promoted catalyst bed. Additional volume of an inert granular material of the specific gravity under reaction conditions below that of the catalyst, preferably below that of the reaction mixture, can be applied onto the catalyst bed in the quantity from 2% to 10% by volume of the catalyst bed. Alternatively, a weak alkaline anion exchanger of the specific gravity under the reaction conditions below that of the catalyst can be used in the quantity of 5% to 15% by volume of the catalyst bed. That arrangement prevents gradual decrease of the flow rate of the mixture received from the filtration elements system **4** and the reaction mixture received from the filtration elements system **3**, located in the catalyst bed, being a result of accumulation and packing of cation exchanger fines and dust at these elements.

The reactor can be operated being completely filled up with the reaction mixture, as presented at Fig.1, and being under the pressure delivered by a pump which supplies the reaction mixture circulated in the system and fresh raw materials to the bottom part of the reactor. Then, the upper filtration elements system **4** is situated just below the top head of the reactor. The reactor can be alternatively operated under the pressure controlled by the nitrogen supply and then the filtration elements system is situated just below the stable reaction mixture level in the top section of the reactor, as illustrated at Fig.2.

Under the operating conditions the reactor is filled with the catalyst up to the level of 50 - 60% of its total height. That has been marked at Fig.1 and Fig.2 as "the static level". When the process is in rUn, the stream of the raw materials and the reaction mixture slackens and the catalyst bed is brought up to "the operating level". At Fig.1 and Fig.2 there has been shown a half of the bed as compared to its "operating level". The filtration elements **2** and **3** are placed in the top half of the catalyst bed.

The reactor design, the method of charging it and the reaction of bisphenol A production are illustrated in the following example.

### Example

The reactor which has a shape of a cylindrical vertical pressure vessel of the diameter equal to 3200 mm and the total height equal to 11000 mm has been provided with four systems of filtration elements in the form of filtration slot-nozzles of the diameter 108 mm and the height from 400 mm to 800 mm. The slot-nozzles have been introduced to the reactor through nozzles in the reactor heads, as presented at Fig.1.

The system **1** is composed of 9 slot-nozzles **5** 400 mm high located just above the reactor bottom head. The system **2** is composed of 13 slot-nozzles 5 800 mm high located at the level of 4500 mm above the reactor bottom - these have been introduced through the nozzles in the reactor bottom head. The system **3** is composed of 12 slot-nozzles 5 800 mm high located at the level of 5500 mm from the reactor bottom - these have been introduced through the nozzles in the reactor top head. The system **4** is composed of **9** slot-nozzles 5 800 mm high located just below the reactor top head.

The filtration slot-nozzles of the system **2** and the system **3** have been protected from sideways movements by means of sleeves **6** being joint points of horizontal frameworks **7** placed at the levels of 4800 mm and 5800 mm and fixed to the reactor wall **8**.

The reactor is completely filled up with strongly acidic cation exchanger Purolite CT 124 wherein 17% of sulfo groups have been neutralised with dimethylthiazolidine as a promoter. The cation exchanger is subjected to dewatering by washing with phenol until the water content in the phenol effluent from the reactor drops below 1.5% by weight. Due to catalyst dewatering, its beads shrink and the catalyst bed in the reactor reaches the level of 5500 mm under the static conditions, that is with no liquid flow.

The reactor is fed from the bottom by means of a pump through the slot-nozzles **5** of the system **1**. The feed stream is a mixture of phenol with 5.5% by weight of acetone with a flow rate of 55000 kg/hour. That is combined with the reaction mixture circulating in the system in the quantity of 55000 kg/hour. The total flow rate of feed through the nozzles 5 of the system 1 reaches 70000 kg/hour. The reaction mixture stream is continuously withdrawn from the catalyst bed at the rate of 30000 kg/hour through the filtration nozzles **5** of the filtration system **2** and the stream of the reaction mixture at the rate 25000 kg/hour through the nozzles **5** of the filtration system **3**. Both streams are put together and cooled down to the temperature of 333 K, and then after having been mixed with the fresh feedstock at the rate of 15000 kg/hour, at the temperature of 328 K, they are recycled to the reactor through the nozzles **5** of the system **1**. A stream of the post-reaction mixture is evacuated at the rate of 15000 kg/hour at the temperature of 339 K from the top of the reactor through the nozzles **5** of the system **4**. The mixture contains 12% by weight of bisphenol A and 0.20% by weight of byproducts, including 0.15% by weight of bisphenol A o,p-isomer. The selectivity of the reaction of phenol and acetone is above 98%.

## Claims

1. The reactor for running the reaction for bisphenol A obtaining from phenol and acetone at the presence of strongly acidic cation exchanger containing chemically bonded reaction promoter as a catalyst, with the reaction mixture passing upwards through the catalyst bed, which has a form of a vertical cylindrical pressure vessel wherein four systems of filtration elements have been inserted through nozzles in the reactor heads, with two systems (**2**) and (**3**) being under the operation conditions immersed in the upper part of the catalyst bed, while the remaining two systems **(1)** and **(4)** being located at the bottom and the top heads of the reactor vessel, respectively.

2. The reactor according to claim 1 wherein the slot-nozzles (**5**) are employed as filtration elements.

3. The reactor according to claim 1 wherein the filtration elements operated in the upper part of the catalyst bed have been protected against sideways movements by means of sleeves (**6**) which loosely embrace them and which form joint points of a horizontal framework (**7**) linking together all the sleeves and being fixed to the reactor wall (**8**) in a number of points spaced along the reactor perimeter.

4. The reactor according to claim 1 wherein the filtration elements have been arranged uniformly over the reactor cross-sections.

5. The reactor according to claim 2 wherein two systems of the filtration slot-nozzles (**5**) operated in the upper part of the catalyst bed are composed of filtration elements mounted on pipes introduced in the reactor space through nozzles in the reactor head, with two filtration elements installed on every pipe: one at the pipe tip and the other one up to 2 m away from the first element.

6. The reactor according to claim 1 wherein the height-to-diameter ratio is from 2.5:1 to 3.3:1.

7. The reactor according to claims 1 and 2 wherein the number of the filtration slot-nozzles (**5**) in the systems **(2)** and **(3)** has been selected to make at least one nozzle fall to 1 m² of the reactor horizontal cross-section.

8. The reactor according to claim 1 wherein the catalyst bed has been supplemented with an inert granular material of the specific gravity under the reaction conditions below that of the catalyst, preferably below that of the reaction mixture, with the volume of that additional material from 2% to 10% by volume of the catalyst bed.

9. The reactor according to claim 1 wherein the catalyst bed has been supplemented with weak alkaline anion exchanger of the specific gravity under the reaction conditions below that of the catalyst and with the volume of the anion exchanger from 5% to 15% by volume of the catalyst bed.
